# EUROPEAN PATENT APPLICATION

(11) **EP 4 520 284 A1**
(43) Date of publication of application: **12.03.2025**
(21) Application number: 24198804.7
(22) Date of filing: 06.09.2024
(51) Int. Cl.: A61B 18/14, A61B 18/00, A61B 34/20

(54) **EXPANDABLE ELECTRODE ASSEMBLY COMPRISING EXTENDED DISTAL END FOR A MEDICAL CATHETER**

(30) Priority: 08.09.2023 US 202363581475 P; 06.08.2024 US 202418795652
(71) Applicant: Biosense Webster (Israel) Ltd., 2066717 Yokneam (IL)
(72) Inventor: BAR-TAL, Meir, 2066717 Yokneam (IL); PHAM, Cuong, Irvine, 92618 (US); NGUYEN, Thanh, Irvine, 92618 (US); DATTA, Keshava, Irvine, 92618 (US); BERMAN, Dror, Irvine, 92618 (US); QUACH, Kate, Irvine, 92618 (US); RAO, Anand, Irvine, 92618 (US)
(74) Representative: Carpmaels & Ransford LLP

(57) **Abstract**

The disclosed technology includes an end effector for a medical probe. The end effector can comprise an expandable distal end assembly extending along a longitudinal axis. The expandable distal end assembly can extend radially outward from the longitudinal axis and define an outer diameter. The end effector can further comprise a distal tip extending distally from the expandable distal end assembly along the longitudinal axis over a tip length greater than the outer diameter. The distal tip can be fixed longitudinally in relation to the expandable distal end assembly and be deflectable with respect to the longitudinal axis. The tip can comprise an electrode configured to deliver ablative energy to tissue or receive signals from tissue.

## Description

### CROSS-REFERENCE TO RELATED APPLICATION

This application claims, under 35 U.S.C. § 119(e), priority to and the benefit of U.S. Provisional Patent Application No. 63/581,475, filed September 8, 2023 (Attorney Docket No.: BIO6884USPSP1 - 253757.000427), the entire contents of which are incorporated herein by reference.

### FIELD

The present invention relates generally to medical devices, and in particular, expandable catheters configured to deliver ablative energy to tissue or receive signals from tissue.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another. Medical probes may utilize radiofrequency (RF) electrical energy to heat tissue. Some ablation approaches use irreversible electroporation (IRE) to ablate cardiac tissue using nonthermal ablation methods.

Pulmonary vein isolation is a procedure commonly used to treat AF. Physicians typically use at least two catheters to complete a pulmonary vein isolation procedure - one having an expandable array of electrodes, such as a balloon or basket catheter, and a second catheter having a focal tip electrode. The catheters are used in combination to deliver ablative energy to identified areas of the heart, and particularly around the pulmonary vein, to block irregular electrical signals. As will be appreciated, typically only one catheter can be inserted into the heart chamber at a time. Removal and insertion of each catheter extends the total procedure time which can be difficult for physician and patient alike, and make recovery more difficult for the patient. Accordingly, there is a need in the art for devices configured to reduce the total procedure time by completing multiple elements of a pulmonary vein isolation procedure with a single device. This and other issues can be addressed by the technology disclosed herein.

### SUMMARY

The disclosed technology includes an end effector for a medical probe. The end effector can comprise an expandable distal end assembly extending along a longitudinal axis. The expandable distal end assembly can extend radially outward from the longitudinal axis and define an outer diameter. The end effector can further comprise a distal tip extending distally from the expandable distal end assembly along the longitudinal axis over a tip length greater than the outer diameter. The distal tip can be fixed longitudinally in relation to the expandable distal end assembly and be deflectable with respect to the longitudinal axis. The tip can comprise an electrode configured to deliver ablative energy to tissue or receive signals from tissue.

The disclosed technology can include a medical probe comprising an insertion tube having a proximal end and a distal end. The insertion tube can extend along a longitudinal axis. The medical probe can further comprise an expandable distal end assembly disposed at the distal end of the insertion tube. The expandable distal end assembly can extend radially outward from the longitudinal axis. The medical probe can further comprise a distal tip extending distally from the expandable distal end assembly along the longitudinal axis. The distal tip can be fixed longitudinally in relation to the expandable distal end assembly and comprise an electrode configured to deliver ablative energy to tissue or receive signals from tissue.

The disclosed technology can further include an end effector for a medical probe comprising a balloon extending along a longitudinal axis and configured to extend radially outward from the longitudinal axis when inflated. The balloon can comprise a stretchable circuit material and a plurality of electrodes disposed on an outer surface of the balloon. The plurality of electrodes can comprise a conductive ink. These and other advantages of the disclosed technology will become more apparent throughout this disclosure in combination with the drawings.

### BRIEF DESCRIPTION OF THE DRAWINGS

FIG. 1 is a schematic pictorial illustration of a medical system including a medical probe having an end effector with electrodes, in accordance with an embodiment of the disclosed technology;
FIG. 2 is a perspective view of a medical probe and handle, in accordance with the disclosed technology;
FIG. 3 is a side view of an end effector, in accordance with the disclosed technology;
FIG. 4A is a perspective view of the end effector, in accordance with the disclosed technology;
FIG. 4B is another perspective view of the end effector, in accordance with the disclosed technology;
FIG. 5 is a top perspective view of the end effector with a distal electrode removed, in accordance with the disclosed technology; and
FIG. 6 is a section view of the end effector taken along line A-A of FIG. 3, in according with the disclosed technology.

### DETAILED DESCRIPTION

The disclosed technology can help to reduce the time required to complete ablation of cardiac tissue, including pulmonary vein isolation procedures, by including an expandable distal end assembly having a plurality of electrodes and a distal tip that extends from the expandable assembly and comprises an electrode. The electrodes on the expandable assembly as well as the electrode on the distal tip can each be configured to deliver ablative energy to tissue or receive signals from tissue. In this way, the disclosed technology combines the functionality of typical balloon or basket ablation catheters with the functionality of a focal ablation catheter, thereby eliminating the need for using multiple catheters to complete pulmonary vein isolation. Stated otherwise, the disclosed technology is capable of performing ablation of both large and small areas of tissue with a single catheter.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician" or "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "tubular" and "tube" are to be construed broadly and are not limited to a structure that is a right cylinder or strictly circumferential in cross-section or of a uniform cross-section throughout its length. For example, although the tubular structures may be generally illustrated as a substantially right cylindrical structure, the tubular structures may have a tapered or curved outer surface without departing from the scope of the present disclosure.

The disclosed technology can be configured to deliver monophasic or biphasic pulses to ablate tissue. For example, the electrodes described herein configured to deliver ablative energy to tissue or receive signals from tissue can be configured to deliver monophasic pulses, biphasic pulses, or some combination thereof. The terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

Example systems, methods, and devices of the present disclosure may be particularly suited for IRE ablation of cardiac tissue to treat cardiac arrhythmias. Ablative energies are typically provided to cardiac tissue by electrodes which can deliver ablative energy alongside the tissue to be ablated. Ablative procedures incorporating such example catheters can be visualized using fluoroscopy, magnetic-based position sensing, and/or active current location techniques.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

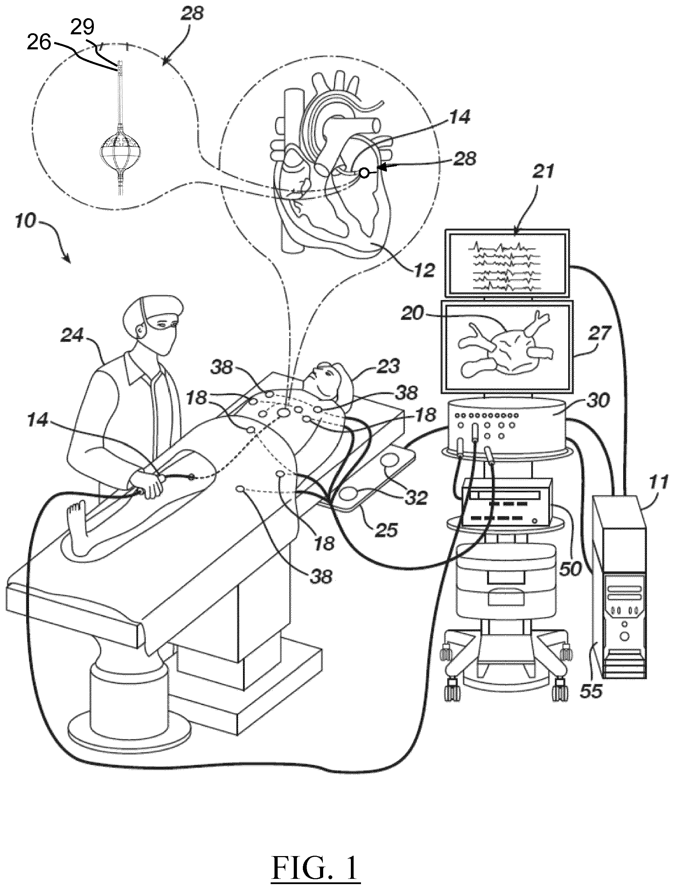

Reference is made to FIG. 1 showing an example catheter-based electrophysiology mapping and ablation system 10. System 10 includes one or more catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The one or more catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. For ablation, physician 24 brings end effector 28 comprising ablation electrodes to a target site for ablating. If the end effector 28 is alternatively or additionally configured for mapping of electrophysiological signals, physician 24 similarly brings the end effector 28 into contact with the heart wall for sensing a target site in heart 12.

Catheter 14 is an exemplary catheter that includes an end effector 28 comprising one and preferably multiple electrodes optionally distributed over an expandable assembly and a distal tip of end effector 28 and configured to deliver ablative energy to tissue or receive signals from tissue. Catheter 14 may additionally include a magnetic-based position sensor 29 embedded in or near end effector 28 for tracking position and orientation of end effector 28. The end effector 28 can further include one or more impedance-based electrodes 26 disposed in or near end effector 28 for tracking position and orientation of end effector 28.

Magnetic-based position sensor 29 may be operated together with a location pad 25 including a plurality of magnetic coils 32 configured to generate magnetic fields in a predefined working volume. Real time position of end effector 28 of catheter 14 may be tracked based on magnetic fields generated with location pad 25 and sensed by magnetic-based position sensor 29. The magnetic-based position sensor 29 can be a single axis sensor, a dual axis sensor, or a triple axis sensor depending on the particular configuration. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091, each of which is incorporated herein by reference as if set forth fully herein.

System 10 includes one or more electrode patches 38 positioned for skin contact on patient 23 to establish location reference for location pad 25 as well as tracking of impedance-based electrodes 26. For impedance-based tracking, electrical current is directed toward impedance-based electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which is incorporated herein by reference as if set forth fully herein.

A recorder 11 displays electrograms 21 captured with body surface ECG electrodes 18 and intracardiac electrograms (IEGM) captured with electrodes of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes disposed on the end effector and configured for delivering ablative energy to tissue. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

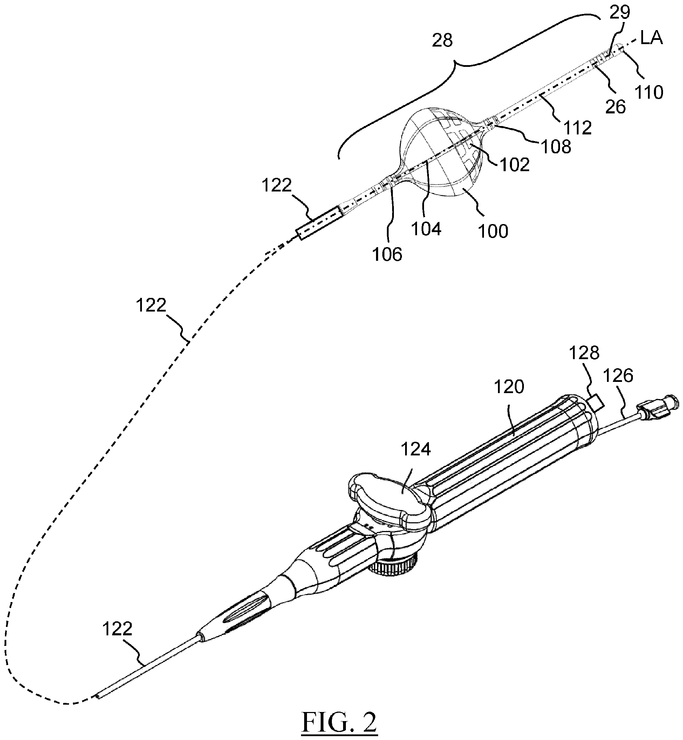

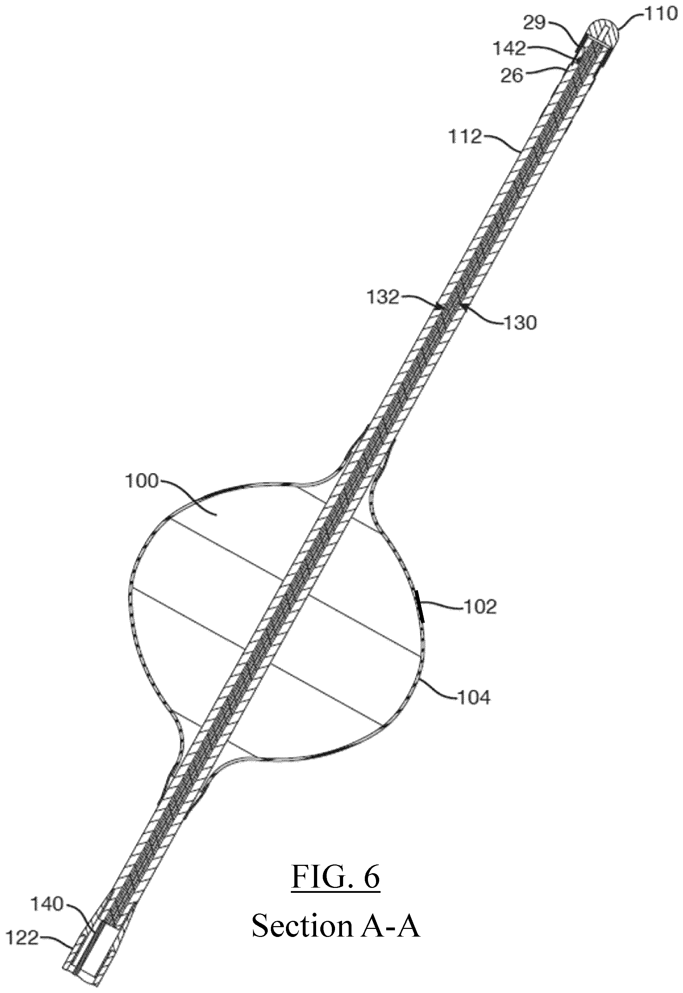

Turning now to FIG. 2, the disclosed technology includes an end effector 28 comprising an expandable distal end assembly 100 and a distal tip 112. The end effector 28 can be attached to a handle 120 by an insertion tube 122 that is configured for insertion through a delivery sheath and/or into vasculature of the patient 23. The handle 120 can include an actuator 124 configured to cause the end effector 28 to deflect radially outward from a longitudinal axis LA. The actuator 124 can be attached to one or more pull wires 140 (as shown in FIG. 6) that are attached to an anchor point 142 disposed near the distal end of the end effector 28. As will be appreciated, the actuator 124 can pull on the pull wire 140 and by pulling on the pull wire 140 the end effector 28 can be caused to deflect radially outward from the longitudinal axis LA.

The handle 120 can further include one or more irrigation supply connectors 126 and one or more electrical connectors 128. The irrigation supply connector 126 can be configured for attachment to an irrigation supply which can deliver irrigation to and through the end effector 28. The electrical connectors 128 can be configured to electrically connect electrodes disposed on the end effector 28 to the PIU 30.

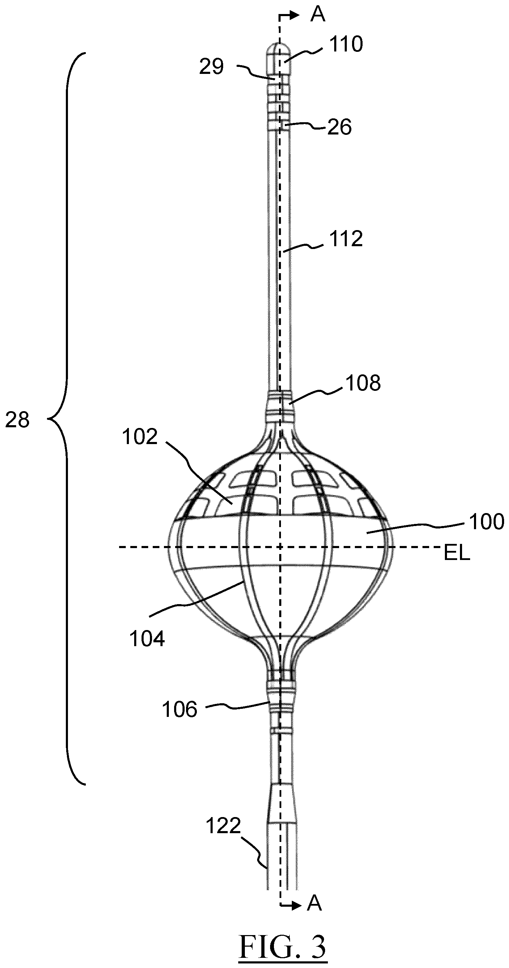

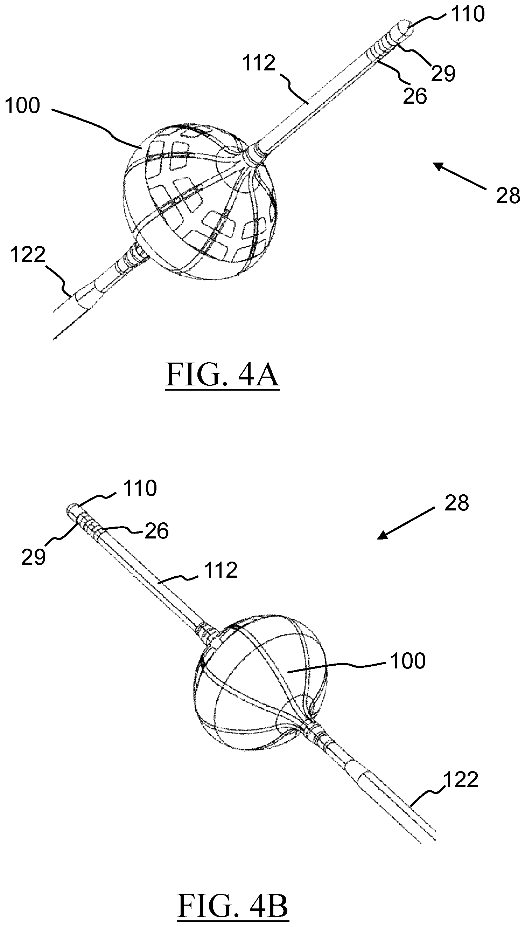

As shown more clearly in FIG. 3, the end effector 28 can include an expandable distal end assembly 100 and a distal tip 112. FIGs. 4A and 4B illustrate alternative perspective views of the end effector 28. The expandable distal end assembly 100 can comprise a plurality of electrodes 102 (only one electrode 102 is labeled for simplicity) disposed around a circumference of the expandable distal end assembly 100 and a plurality of electrical traces 104 disposed along the expandable distal end assembly 100 with each electrical trace 104 electrically connected to one or more electrodes 102. The electrodes 102 can each be disposed on a distal half of the expandable distal end assembly 100. Stated otherwise, the electrodes 102 can each be disposed on a distal side of an equatorial line EL (in the, upper portion of Fig. 3) of the expandable distal end assembly 100. In this way, the electrodes 102 can be positioned to deliver ablative energy to tissue or receive signals from tissue on or around the pulmonary vein when the expandable distal end assembly 100 is inserted at least partially into a pulmonary vein. A proximal coupler 106 can couple the expandable distal end assembly 100 to the insertion tube 122 and a distal coupler 108 can couple the expandable distal end assembly 100 to a distal tip 112.

The electrodes 102 can be any type of electrode including, but not limited to, electrodes suitable for sensing electrophysiological signals, delivering ablative energy to tissue, position sensing, reference electrodes, etc. The electrodes 102 can be any shape and formed of conductive material such as gold, silver, platinum, iridium, etc. The electrodes 102 can be configured to deliver electrical pulses for irreversible electroporation, the pulses having a peak voltage of at least 900 volts (V).

The expandable distal end assembly 100 can be configured to transition between an expanded configuration (as shown in FIG. 2) and a collapsed configuration (now shown) in which the expandable distal end assembly 100 is collapsed and configured to be pulled or pushed through an insertion sheath and/or vasculature of patient 23. The expandable distal end assembly 100 can, for example, be an inflatable balloon configured to be inflated to transition to the expanded configuration. The balloon can be inflated by irrigation fluid received through the irrigation supply connector 126. In some examples, the expandable distal end assembly 100 can comprise one or more apertures (not shown) configured to permit the irrigation fluid to exit the expandable distal end assembly 100. Alternatively, the expandable distal end assembly 100 can be configured to be inflated by the irrigation fluid but be sealed or at least partially sealed so that no irrigation fluid escapes through a wall of the expandable distal end assembly 100. In this configuration, the irrigation fluid can be delivered to the distal tip 112 and/or through the distal tip 112. Alternatively, the expandable distal end assembly 100 and the distal tip 112 can each have apertures and be configured to permit irrigation fluid to flow therethrough.

The expandable distal end assembly 100 can be made from a stretchable circuit material and the electrodes 102 and/or electrical traces 104 can be printed directly onto the expandable distal end assembly 100 using conductive ink without the need for a polyimide substrate material. The expandable distal end assembly 100, for example, can be made from Beyolex^{™} made by Panasonic^{™}, or other similar stretchable circuit material. In other examples, the electrodes 102 and/or electrical traces 104 can be printed onto stretchable circuit material to form a flexible circuit and the flexible circuit can be disposed along an outer surface of the expandable distal end assembly 100.

Although not shown, it will be appreciated that the expandable distal end assembly 100 can alternatively comprise a plurality of spines configured to bow radially outward from the longitudinal axis LA to form a basket assembly. The electrodes 102 can be disposed along the spines and the basket assembly can be configured to transition between an expanded configuration when pushed out of a delivery sheath and a collapsed configuration when disposed in the delivery sheath.

Distal tip 112 can extend distally from the expandable distal end assembly 100 along the longitudinal axis LA. The distal tip 112 can extend distally from the expandable distal end assembly 100 over a tip length that is greater than an outer diameter of the expandable distal end assembly 100. For example, the expandable distal end assembly 100 can have a diameter measured at its equatorial line EL, or a location where the diameter of the expandable distal end assembly 100 is greatest, and the tip length of the distal tip 112 can be greater than the length of the diameter. In some examples, the length of the distal tip 112 can be one and a half, two, three, four, or even at least five times greater than the diameter of the expandable distal end assembly 100. By having a distal tip 112 that is elongated as shown, the distal tip 112 can be configured to deflect radially outward from the longitudinal axis LA when the pull wire 140 is pulled by the actuator 124 as previously described. The distal tip 112 can be fixed longitudinally in relation to the expandable distal end assembly 100.

The distal tip 112 can comprise a distal electrode 110 that is disposed on a distal end of the distal tip 112. The distal electrode 110 can be configured to deliver ablative energy to tissue or receive signals from tissue. By including a distal electrode 110 disposed on the distal end of the distal tip 112, the distal electrode 110 can be configured to be used as a focal electrode for delivering ablative energy to small or difficult to reach regions of the heart 12. Thus, the end effector 28 can combine the benefits of having multiple electrodes 102 disposed on an expandable distal end assembly 100 that can be configured to deliver ablative energy to a relatively large area of tissue (e.g., around the pulmonary vein) with the benefit of having a focal electrode (distal electrode 110) configured to deliver ablative energy to small areas of tissue. In this way, the disclosed technology reduces or altogether eliminates the need for using multiple catheters during a procedure, thereby helping to reduce the total procedure time. The distal electrode 110 can be configured to deliver electrical pulses for irreversible electroporation, the pulses having a peak voltage of at least 900 volts (V).

To help determine a position and orientation of the distal tip 112, the distal tip 112 can have one or more magnetic-based position sensors 29 disposed near the distal end of the distal tip 112. As described previously, the magnetic-based position sensors 29 can be configured to generate a current when subjected to a magnetic field generated by magnetic coils 32. Alternatively, or in addition, the distal tip 112 can have one or more impedance-based electrodes 26 disposed thereon and configured to detect a position and orientation of the distal tip 112 using active current location as previously described. Accordingly, the end effector 28 can have one or more position sensors configured to detect a position and orientation of the end effector 28 which can then be used to render on a display a representation of the location and orientation of the end effector 28.

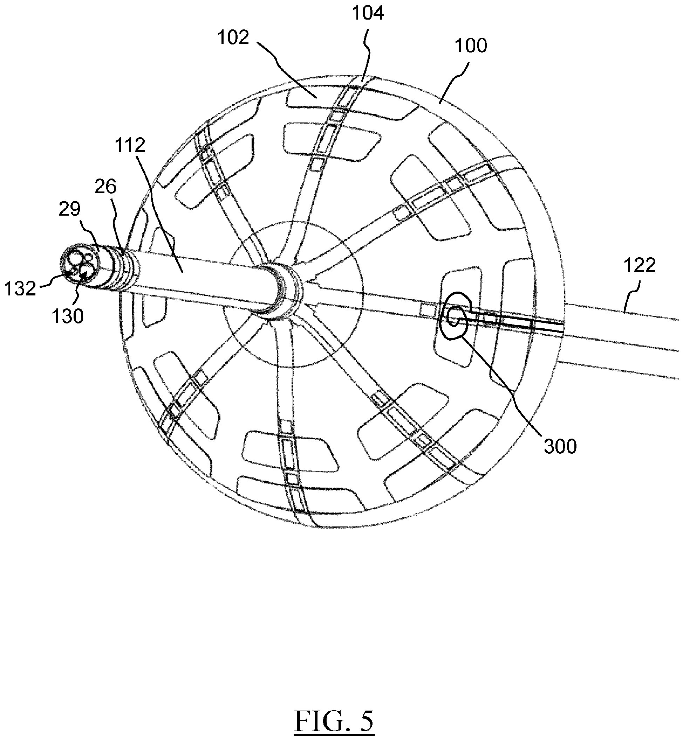

FIG. 5 illustrates a perspective view of the end effector 28 with the distal electrode 110 removed to show large lumens 130 and small lumens 132 extending through the distal tip 112. As will be appreciated, the large lumens 130 and the small lumens 132 can extend all the way through the insertion tube 122 and to the handle 120. The large lumens 130 can be configured to receive irrigation fluid and/or a pull wire 140 can be disposed through at least one of the large lumens 130. In this way, irrigation fluid can be delivered to the distal electrode 110. Although not show, the distal electrode 110 can have one or more apertures formed therethrough configured to deliver irrigation fluid therethrough. Alternatively, or in addition, the distal tip 112 can have one or more apertures formed therethrough configured to deliver irrigation fluid therethrough. Referring back to Fig. 5, it can be seen the location of each of the electrode and therefore the overall shape of the basket can be determined via the use of a magnetic field sensor 300 in the form of a flat loop by a single wire with two leads running along the balloon (inside or outside) back to the handle. Each electrode would have the wire loop in the form of a helix so that the location of each electrode can be visualized. Support for the magnetic location sensor 300 and the shape determination of this sensor can be understood from U.S. Patent Publication Nos. US20180344202A1, US20220265194A1 or U.S. Patent No. US10687761B2 which are each incorporated by reference with a copy provided in the appendix of priority application, U.S. Provisional Patent Application No. 63/581,475.

The small lumens 132 can be configured to house one or more electrical wires that can extend from the handle 120 to the distal electrode 110, the magnetic-based position sensor 29, or the impedance-based electrodes 26. As will be appreciated, the number of large lumens 130 and small lumens 132 can be greater or fewer than those shown depending on the particular configuration. Furthermore, although the large lumens 130 are described as being configured to receive irrigation fluid or house a pull wire 140 and the small lumens 132 are described as configured to house electrical wire, it will be appreciated that one or more small lumens 132 can similarly be configured to receive irrigation fluid and/or house a pull wire 140 and one or more large lumens 130 can be configured to house electrical wire depending on the particular configuration.

As shown in FIG. 6, which is a section view of the end effector 28 taken along line A-A of FIG. 3, a pull wire 140 can extend through at least one of the large lumens 130 and be attached to an anchor point 142 disposed near the distal electrode 110. As previously described, as the actuator 124 is actuated, the pull wire can pull on the distal tip 112 can cause the distal tip 112 to deflect radially outward from the longitudinal axis LA. Although not shown, it will be appreciated that the end effector can include more than one pull wire 140 and the anchor points 142 can be disposed in various locations to affect the radius of articulation of the distal tip 112 and/or the entire end effector 28.

The disclosed technology described herein can be further understood according to the following clauses:
Clause 1: An end effector for a medical probe comprising: an expandable distal end assembly extending along a longitudinal axis, the expandable distal end assembly extending radially outward from the longitudinal axis and defining an outer diameter; and a distal tip extending distally from the expandable distal end assembly along the longitudinal axis over a tip length greater than the outer diameter, the distal tip being fixed longitudinally in relation to the expandable distal end assembly and deflectable with respect to the longitudinal axis, the tip comprising an electrode configured to deliver ablative energy to tissue or receive signals from tissue.
Clause 2: The end effector of any of clause 1, the expandable distal end assembly further comprising a plurality of electrodes disposed on the expandable distal end assembly and configured to deliver ablative energy to tissue or receive signals from tissue.
Clause 3: The end effector of clause 2, the plurality of electrodes being disposed radially along a circumference of the expandable distal end assembly distal to an equatorial line of the expandable distal end assembly.
Clause 4: The end effector of any of the preceding clauses, the distal tip further configured to deflect radially outward from the longitudinal axis.
Clause 5: The end effector of clause 4 further comprising a pull wire configured to cause the distal tip to deflect radially outward from the longitudinal axis.
Clause 6: The end effector of any of the preceding clauses, the distal tip extending distally from the expandable distal end assembly a distance of at least one and a half times a length of the outer diameter of the expandable distal end assembly.
Clause 7: The end effector of clause 6, the distance comprising two times the length of the outer diameter of the expandable distal end assembly.
Clause 8: The end effector of any of the preceding clauses, the distal tip further comprising an impedance-based position sensor.
Clause 9: The end effector of any of the preceding clauses, the distal tip further comprising an electromagnetic position sensor configured to output a current when subjected to a magnetic field generated by a magnetic field generator.
Clause 10: The end effector of clause 9, the electromagnetic position sensor comprising a triple axis sensor.
Clause 11: The end effector of any of the preceding clauses, the end effector further configured to permit irrigation fluid to flow through at least the distal tip.
Clause 12: The end effector of any of the preceding clauses, the expandable distal end assembly configured to transition between an expanded configuration and a collapsed configuration.
Clause 13: The end effector of clause 12, the expandable distal end assembly comprising a balloon configured to be inflated and deflated to transition the expandable distal end assembly between the expanded configuration and the collapsed configuration.
Clause 14: The end effector of clause 13, the expandable distal end assembly being configured to be inflated by receiving irrigation fluid, the expandable distal end assembly being sealed to prevent irrigation fluid from exiting the expandable distal end assembly.
Clause 15: The end effector of clause 13 or clause 14, the balloon comprising a stretchable circuit material.
Clause 16: The end effector of clause 15, the plurality of electrodes being printed directly on the stretchable circuit material.
Clause 17: The end effector of clause 16, the plurality of electrodes comprising a conductive ink.
Clause 18: The end effector of clause 13, the balloon comprising a flexible circuit disposed on an outer surface of the balloon, the flexible circuit being electrically connected to at least some of the plurality of electrodes.
Clause 19: The end effector of clause 12, the expandable distal end assembly comprising a plurality of spines configured to bow radially outward from the longitudinal axis to transition the distal end assembly between the expanded configuration and the collapsed configuration.
Clause 20: A medical probe, comprising: an insertion tube having a proximal end and a distal end, the insertion tube extending along a longitudinal axis; an expandable distal end assembly disposed at the distal end of the insertion tube, the expandable distal end assembly extending radially outward from the longitudinal axis; and a distal tip extending distally from the expandable distal end assembly along the longitudinal axis, the distal tip being fixed longitudinally in relation to the expandable distal end assembly and comprising an electrode configured to deliver ablative energy to tissue or receive signals from tissue.
Clause 21: The medical probe of clause 20, the distal tip further configured to deflect radially outward from the longitudinal axis.
Clause 22: The medical probe of clause 21 further comprising a pull wire configured to cause the distal tip to deflect radially outward from the longitudinal axis.
Clause 23: The medical probe of clause 22 further comprising a handle disposed at the proximal end of the insertion tube, the handle comprising an actuator configured to pull the pull wire to cause the distal tip to deflect radially outward from the longitudinal axis.
Clause 24: The medical probe of any of clauses 20-23, the distal tip extending distally from the expandable distal end assembly a distance of at least one and a half times a length of a diameter of the expandable distal end assembly.
Clause 25: The medical probe of clause 24, the distance comprising two times the length of the diameter of the expandable distal end assembly.
Clause 26: The medical probe of any of clauses 20-25, the distal tip further comprising an impedance-based position sensor.
Clause 27: The medical probe of any of clauses 20-26, the distal tip further comprising an electromagnetic position sensor configured to output a current when subjected to a magnetic field generated by a magnetic field generator.
Clause 28: The medical probe of clause 27, the electromagnetic position sensor comprising a triple axis sensor.
Clause 29: The medical probe of any of clauses 20-28, the medical probe further configured to permit irrigation fluid to flow through at least the distal tip.
Clause 30: The medical probe of any of clauses 20-29, the expandable distal end assembly further comprising a plurality of electrodes disposed on the expandable distal end assembly and configured to deliver ablative energy to tissue or receive signals from tissue.
Clause 31: The medical probe of clause 30, the plurality of electrodes being disposed radially along a circumference of the expandable distal end assembly.
Clause 32: The medical probe of any of clauses 20-31, the expandable distal end assembly configured to transition between an expanded configuration and a collapsed configuration.
Clause 33: The medical probe of clause 32, the expandable distal end assembly comprising a balloon configured to be inflated and deflated to transition the expandable distal end assembly between the expanded configuration and the collapsed configuration.
Clause 34: The medical probe of clause 33, the balloon comprising a stretchable circuit material.
Clause 35: The medical probe of clause 34, the plurality of electrodes being printed directly on the stretchable circuit material.
Clause 36: The medical probe of clause 35, the plurality of electrodes comprising a conductive ink.
Clause 37: The medical probe of clause 33, the balloon comprising a flexible circuit disposed on an outer surface of the balloon, the flexible circuit being electrically connected to at least some of the plurality of electrodes.
Clause 38: The medical probe of clause 32, the expandable distal end assembly comprising a plurality of spines configured to bow radially outward from the longitudinal axis to transition the distal end assembly between the expanded configuration and the collapsed configuration.
Clause 39: An end effector for a medical probe comprising: a balloon extending along a longitudinal axis and configured to extend radially outward from the longitudinal axis when inflated, the balloon comprising a stretchable circuit material; and a plurality of electrodes disposed on an outer surface of the balloon, the plurality of electrodes comprising a conductive ink.
Clause 40: The end effector of clause 39 further comprising a plurality of flexible traces printed on the outer surface of the balloon using a conductive ink, the plurality of flexible traces being electrically connected to the plurality of electrodes.
Clause 41: The end effector of clause 39 or clause 40, further comprising a distal tip extending from a distal end of the balloon, the distal tip comprising an electrode configured to deliver ablative energy to tissue or receive signals from tissue.
Clause 42: The end effector of any of clauses 39-41, the distal tip further configured to deflect radially outward from the longitudinal axis.
Clause 43: The end effector of clause 42 further comprising a pull wire configured to cause the distal tip to deflect radially outward from the longitudinal axis.
Clause 44: The end effector of any of clauses 39-43, the distal tip extending distally from the balloon a distance of at least one and a half times a length of a diameter of the balloon.
Clause 45: The end effector of clause 44, the distance comprising two times the length of the diameter of the balloon.
Clause 46: The end effector of any of clauses 39-45, the distal tip further comprising an impedance-based position sensor.
Clause 47: The end effector of any of clauses 39-46, the distal tip further comprising an electromagnetic position sensor configured to output a current when subjected to a magnetic field generated by a magnetic field generator.
Clause 48: The end effector of clause 47, the electromagnetic position sensor comprising a triple axis sensor.
Clause 49: The end effector of any of clauses 39-48, the end effector further configured to permit irrigation fluid to flow through at least the distal tip.
Clause 50: The end effector of any of clauses 39-49, the plurality of electrodes being disposed radially along a circumference of the balloon.
Clause 51: The end effector of any of clauses 39-50, the balloon configured to transition between an expanded configuration and a collapsed configuration.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. An end effector for a medical probe comprising:
an expandable distal end assembly extending along a longitudinal axis, the expandable distal end assembly extending radially outward from the longitudinal axis and defining an outer diameter; and
a distal tip extending distally from the expandable distal end assembly along the longitudinal axis over a tip length greater than the outer diameter, the distal tip being fixed longitudinally in relation to the expandable distal end assembly and deflectable with respect to the longitudinal axis, the distal tip comprising an electrode configured to deliver ablative energy to tissue or receive electrophysiological signals from tissue.

2. The end effector of claim 1, the expandable distal end assembly further comprising a plurality of electrodes disposed on the expandable distal end assembly and configured to deliver ablative energy to tissue or receive signals from tissue, optionally the plurality of electrodes being disposed radially along a circumference of the expandable distal end assembly distal to an equatorial line of the expandable distal end assembly.

3. The end effector of claim 1, the distal tip further comprising an impedance-based position sensor.

4. The end effector of claim 1, the distal tip further comprising an electromagnetic position sensor configured to output a current when subjected to a magnetic field generated by a magnetic field generator, optionally the electromagnetic position sensor comprising a triple axis sensor.

5. The end effector of claim 1, the end effector further configured to permit irrigation fluid to flow through at least the distal tip.

6. The end effector of claim 1, the expandable distal end assembly comprising a balloon configured to be inflated and deflated to transition the expandable distal end assembly between an expanded configuration and a collapsed configuration.

7. The end effector of claim 6, the balloon comprising a stretchable circuit material.

8. The end effector of claim 7, the balloon comprising a plurality of electrodes being printed directly on the stretchable circuit material.

9. The end effector of claim 8, the plurality of electrodes comprising a conductive ink, optionally the balloon comprising a flexible circuit disposed on an outer surface of the balloon, the flexible circuit being electrically connected to at least some of the plurality of electrodes.

10. A medical probe, comprising:
an insertion tube having a proximal end and a distal end, the insertion tube extending along a longitudinal axis;
an expandable distal end assembly disposed at the distal end of the insertion tube, the expandable distal end assembly extending radially outward from the longitudinal axis; and
a distal tip extending distally from the expandable distal end assembly along the longitudinal axis, the distal tip being fixed longitudinally in relation to the expandable distal end assembly and comprising an electrode configured to deliver ablative energy to tissue or receive signals from tissue.

11. The end effector of claim 1 or the medical probe of claim 10, the distal tip further configured to deflect radially outward from the longitudinal axis.

12. The end effector of claim 1 or the medical probe of claim 10, the distal tip extending distally from the expandable distal end assembly a distance of at least one and a half times a length of a diameter of the expandable distal end assembly.

13. The medical probe of claim 10, the expandable distal end assembly further comprising a stretchable circuit material and a plurality of electrodes, the plurality of electrodes being printed directly on the stretchable circuit material.

14. An end effector for a medical probe comprising:
a balloon extending along a longitudinal axis and configured to extend radially outward from the longitudinal axis when inflated, the balloon comprising a stretchable circuit material; and
a plurality of electrodes disposed on an outer surface of the balloon, the plurality of electrodes comprising a conductive ink.

15. The end effector of claim 14 further comprising a plurality of flexible traces printed on the outer surface of the balloon using a conductive ink, the plurality of flexible traces being electrically connected to the plurality of electrodes.
